# EUROPEAN PATENT APPLICATION

(11) **EP 3 335 618 A2**
(43) Date of publication of application: **20.06.2018**
(21) Application number: 17205479.3
(22) Date of filing: 05.12.2017
(51) Int. Cl.: A61B 1/00, A61B 1/05, A61B 1/307, A61B 1/005, A61B 1/06

(54) **IMAGING MINI-SCOPE FOR ENDOSCOPE SYSTEM**

(30) Priority: 13.12.2016 US 201662433467 P
(71) Applicant: Cook Medical Technologies LLC, Bloomington, IN 47404 (US)
(72) Inventor: BRUNCH, Kristin M., Bloomington, IN Indiana 47408 (US); MISKEWYCZ, Darian, Bloomington, IN Indiana 47401 (US); SMITH, Johnny P., Worthington, IN Indiana 47471 (US)
(74) Representative: Garratt, Peter Douglas

(57) **Abstract**

An imaging mini-scope (14) for use with an endoscope system (10) includes an elongated body (40) having a first length. The elongated body includes at least one passage (46) extending along the first length. A flexible distal tip portion (80) is coupled to the elongated body. An imaging device (60) includes an imaging sensor (62) disposed at the flexible distal tip portion and a signal transmission connection (64) extending through the at least one passage to connect the imaging sensor to a processing system. The imaging sensor is configured to detect image information and the signal transmission connection is configured to transmit one or more signals indicative of the detected image information from the imaging sensor to the processing system. A light source (70) is disposed at the flexible distal tip portion. The light source extends through the at least one passage.

## Description

### BACKGROUND

Access sheaths, such as ureteral access sheaths, may be used to gain access to body cavities in lumens during endoscopic and laparoscopic surgery, and by other procedures that generally use minimally invasive techniques. Ureteral access sheaths may be used with an endoscope for finding and removing kidney stones, and may be used in other applications, such as access to bile ducts, tissue biopsy and removal, and diagnostic visualization, for example. Other applications for which an access sheath has been used include vascular procedures, as well as procedures requiring gastro-intestinal access, uterine access, and bronchial access, for example. At least some endoscopes, hysteroscopes, sigmoidoscopes, bronchoscopes, and other types of instruments for minimally-invasive techniques may include such access sheaths.

A sheath may be utilized during a medical procedure to protect the tissues of a patient. For example, to remove a kidney stone, conventional procedures may require repeated introduction and removal of a retrieval basket across a patient's ureter to remove stone fragments. Passing the retrieval basket through the access sheath instead of through the ureter itself avoids trauma to the ureter and the surrounding tissues. With an access sheath providing access across a ureter, the surgeon may wish to use the access sheath for access not only for an endoscope, but also for multiple endoscopic instruments, such as a retrieval basket, a stone "blocker" or back stop, a fiber optic laser to break up stones, a safety wire, an operating wire, or a system to provide irrigation or to instill contrast agents. While all of these systems are desirable, it is difficult to operate them all at the same time and through a common access sheath. As a result, the surgeon may also pass instruments through the endoscope as well as the access sheath.

Removal of kidney stones and other calculi within body cavities may be accomplished with an endoscope. The endoscope is inserted into the patient through a body passageway, such as the ureter. The endoscope includes an integral optical system, a working channel, and a controller to maneuver the endoscope so that the surgeon can accomplish a therapeutic or diagnostic procedure. The surgeon positions the endoscope so that the surgeon can observe the desired body part of the patient using the optical system, with irrigation if necessary. The surgeon then uses at least one instrument, such as a laser or a grasper, to break up and remove objects in the body passageway. The endoscope may also be used for diagnostic purposes, such as for observing the desired portion of the patient and then taking a biopsy sample.

Effective diagnostic visualization, particularly, in small passages or spaces, before and/or during endoscopic and laparoscopic surgery including an increased ability to navigate through tortuous body passageways and cavities while allowing for important access functions continues to be a priority.

### SUMMARY

In one example embodiment, an imaging mini-scope for use with an endoscope system includes an elongated body having a first length. The elongated body includes at least one passage extending along the first length. A flexible distal tip portion is coupled to the elongated body. An imaging device includes an imaging sensor disposed at the flexible distal tip portion and a signal transmission connection extending through the at least one passage to connect the imaging sensor to a processing system. The imaging sensor is configured to detect image information and the signal transmission connection is configured to transmit one or more signals indicative of the detected image information from the imaging sensor to the processing system. A light source is disposed at the flexible distal tip portion. The light source extends through the at least one passage.

In another example embodiment, a system includes an access sheath having a channel extending along a first length of the access sheath between a first distal end and an opposing first proximal end. A dilator is disposed at the first distal end. The dilator includes a passage coaxial with the channel and a longitudinal slit intersecting the passage. An imaging mini-scope is at least partially positioned in the passage and removable from the passage through the longitudinal slit. The imaging mini-scope includes an elongated body having at least one passage extending along a second length of the elongated body between a second distal end and an opposing second proximal end. An imaging device extends through the at least one passage. The imaging device includes an imaging sensor disposed at the second distal end and a signal transmission connection coupled to the imaging sensor. A light source extends through the at least one passage. The light source is configured to emit light at the second distal end.

In another example embodiment, a method for introducing an imaging mini-scope into a lumen of a human includes removably coupling an imaging mini-scope to a distal end of an access sheath having a channel extending along a length of the access sheath between the distal end and an opposing proximal end, introducing the access sheath and the imaging mini-scope into the lumen, navigating the access sheath and the imaging mini-scope to a target location, and removing the imaging mini-scope from the access sheath.

### BRIEF DESCRIPTION OF THE DRAWINGS

The detailed description is set forth with reference to the accompanying figures. The use of the same reference numbers in different figures indicates similar or identical items or features.
FIG. 1 is a perspective view of an example system including an access sheath and an imaging mini-scope removably coupled to the access sheath, according to various embodiments;
FIG. 2 is a perspective view of a portion of an example system including an imaging mini-scope removably coupled to the access sheath, according to various embodiments;
FIG. 3 is a side view of an example imaging mini-scope having a flexible distal tip portion in a first position, according to various embodiments;
FIG. 4 is a sectional view of the example imaging mini-scope shown in FIG. 3;
FIG. 5 is a side view of the example imaging mini-scope shown in FIG. 3 having a flexible distal tip portion in a deflected position, according to various embodiments;
FIG. 6 is a sectional side view of an example imaging mini-scope with a flexible distal tip portion, according to various embodiments;
FIG. 7 is a perspective view of an example deflection band for an imaging mini-scope with a flexible distal tip portion, according to various embodiments;
FIG. 8 is a side view of an example deflection band for an imaging mini-scope with a flexible distal tip portion and a rigid elongated body, according to various embodiments;
FIG. 9 illustrates an example method for introducing an imaging mini-scope into a lumen of a human, according to various embodiments; and
FIGS. 10 and 11 are schematic views of a portion of a system including an access sheath and an imaging mini-scope removably coupled to the access sheath during various steps of the example method illustrated in FIG. 9.

### DETAILED DESCRIPTION

Example embodiments of the present invention are disclosed herein. It is understood, however, that the disclosed embodiments are merely exemplary and may be embodied in various and alternative forms. The figures are not necessarily to scale; some figures may be configured to show the details of a particular component. Therefore, specific structural and functional details disclosed herein are not to be interpreted as limiting but merely as a representative basis for the claims and/or teaching one skilled in the art to practice the embodiments.

Example embodiments seek to overcome some of the concerns associated with visualization of body pathways and cavities, which may be tortuous, during endoscopic and laparoscopic surgery. Example embodiments describe a system including an access sheath, a dilator disposed at a distal end of the access sheath, and an imaging mini-scope removably coupled to the access sheath or dilator that may be used for endoscopic or laparoscopic surgical procedures.

In example embodiments, a system includes an access sheath having a channel extending along a length of the access sheath. A dilator is disposed at the distal end of the access sheath and includes a passage coaxial with the channel and a longitudinal slit intersecting the passage. An imaging mini-scope is at least partially positioned in the passage and removable from the passage through the longitudinal slit. The imaging mini-scope includes an elongated body having a passage extending along a length of the elongated body, and an imaging device including an imaging sensor and a light source disposed at the distal end of the elongated body. The imaging mini-scope as described herein provides for direct vision in the urinary system, for example, in tight spaces that current endoscopes may not be able to access due to size limitations. For example, the imaging mini-scope described herein is able to navigate past a kink or a stricture in the ureter, access tight anatomical areas such as the ureteropelvic junction (the UPJ), the ureterovesical junction (the UVJ), and associated crossing vessels, and navigate tight corners, such as in the superior and inferior poles of the patient's kidney. The example imaging mini-scope and/or the example system may be suitable for other applications including, for example, interventional radiology, aortic intervention or peripheral intervention. The example imaging mini-scope includes features that facilitate navigation through small or tight spaces and deflection and visualization to facilitate access to the target site that conventionally were dependent on the use of fluoroscopy, which increases exposure to radiation. The example imaging mini-scope also includes features to perform ureteroscopy procedures that do not rely on the use of a traditional endoscope, an associated wire guide and/or fluoroscopy.

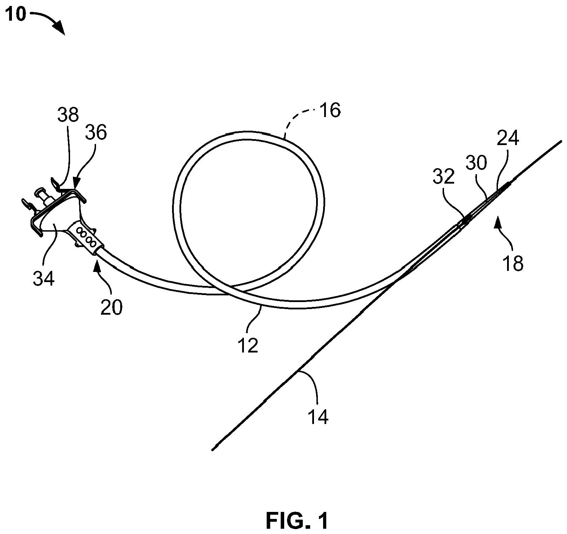

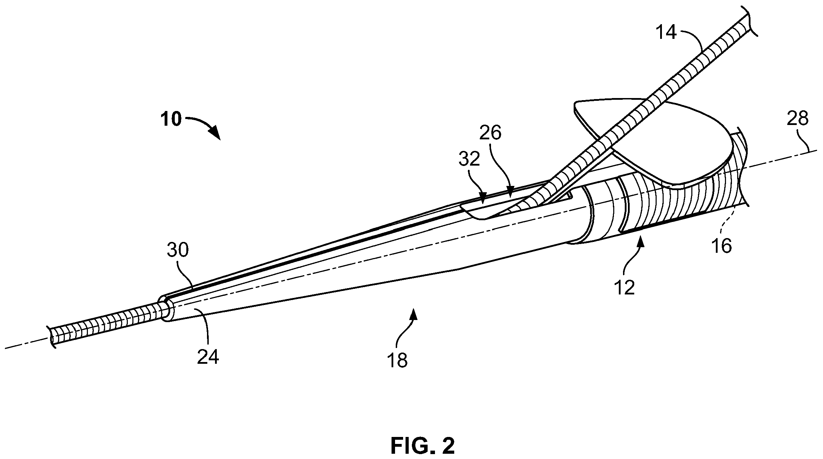

Referring now to the figures, FIG. 1 is a perspective view of an example system 10 including an access sheath 12, e.g., a ureteral access sheath, and an imaging mini-scope 14 removably coupled to access sheath 12. FIG. 2 is a perspective view of a portion of example system 10 including imaging mini-scope 14 removably coupled to access sheath 12. As shown in FIG. 1, in example embodiments, access sheath 12 includes a channel 16 extending along a length of access sheath 12 between a distal end 18 and an opposing proximal end 20. A dilator 24 is disposed at distal end 18 of access sheath 12. Referring further to FIG. 2, dilator 24 includes a passage 26 formed along a length of dilator 24 and coaxial with channel 16. In example embodiments, passage 26 and channel 16 are coaxially aligned along a longitudinal axis 28 of access sheath 12, as shown in FIG. 2. A longitudinal slit 30 extends along at least a portion of a length of dilator 24 and coplanar with longitudinal axis 28 to intersect passage 26.

In example embodiments, imaging mini-scope 14 is removably positionable within passage 26 via longitudinal slit 30. More specifically, in example embodiments, longitudinal slit 30 is sized to allow imaging mini-scope 14 to be urged through longitudinal slit 30 and positioned within passage 26. Further, passage 26 has a suitable inner diameter to allow imaging mini-scope 14 to move within passage 26. Once system 10 is suitably positioned at or near a target site, imaging mini-scope 14 is removable from within passage 26 through longitudinal slit 30 such that imaging mini-scope 14 can be operated and moved independently of access sheath 12. In example embodiments, access sheath 12 is movable toward dilator 24 to urge imaging mini-scope 14 from passage 26 though longitudinal slit 30, as described in more detail below. By removing imaging mini-scope 14 from passage 26, the operator (e.g., the surgeon or doctor) is able to introduce one or more instruments through channel 16 of access sheath 12 and/or passage 26 to the target site without interference from imaging mini-scope 14. In a particular embodiment, longitudinal slit 30 transitions into an opening 32, as shown in FIG. 2, providing communication with passage 26 to facilitate positioning imaging mini-scope 14 within passage 26 and/or removing imaging mini-scope 14 from within passage 26.

In example embodiments, access sheath 12 includes a funnel 34 disposed at proximal end 20 that acts as a handle in certain embodiments during insertion. Funnel 34 includes a trough 36 to facilitate instrument introduction into channel 16 of access sheath 12. A dilator hub 38 is also disposed at proximal end 20. In certain embodiments, dilator hub 38 includes a locking mechanism to secure dilator 24 to access sheath 12 for simultaneous advancement of access sheath 12 and dilator 24 through the patient's lumen toward the target site. In example embodiments, an external surface of access sheath 12 and/or an external surface of dilator 24 have a suitable coating, such as a hydrophilic coating, to create a low-friction surface to ease the insertion and advancement process.

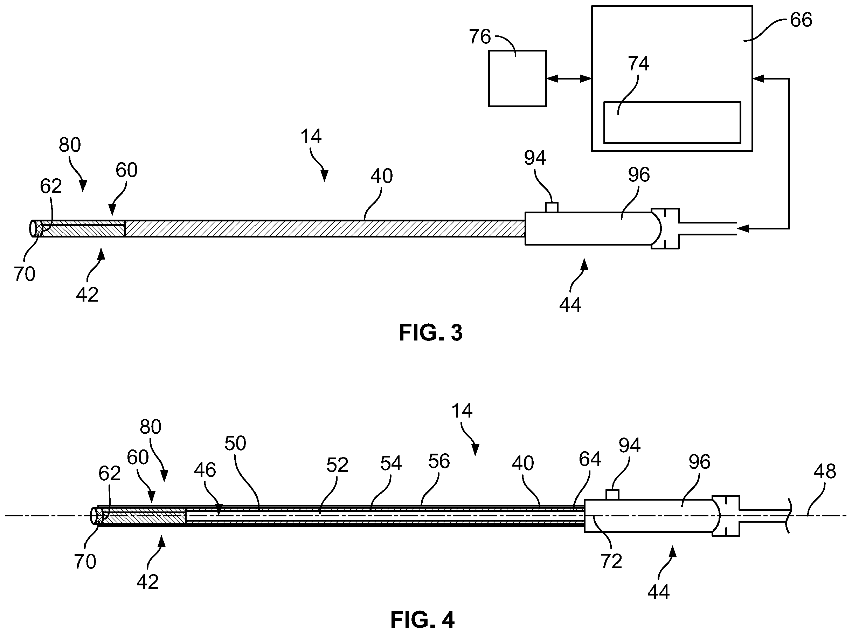

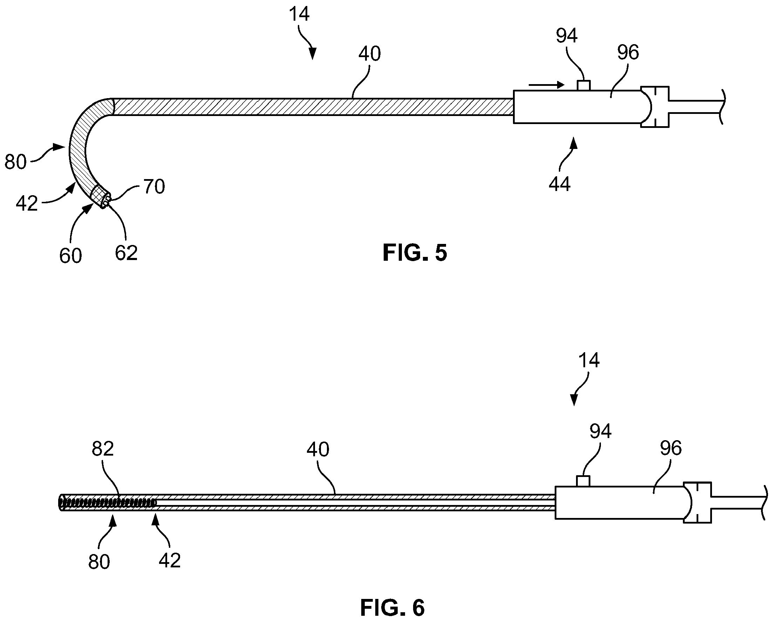

FIG. 3 is a side view of example imaging mini-scope 14 in a first or introduction position, FIG. 4 is a sectional view of example imaging mini-scope 14 shown in FIG. 3, and FIG. 5 is a side view of example imaging mini-scope 14 shown in FIG. 3 in a second or example imaging position having a flexible distal tip portion in a deflected configuration. Referring to FIGS. 3-5, imaging mini-scope 14 includes an elongated body 40 having a distal end 42 and an opposing proximal end 44. Elongated body 40 has a first length that extends between distal end 42 and proximal end 44. Elongated body 40 includes at least one passage 46, as shown in FIG. 4, for example, which extends the first length of elongated body 40 along a longitudinal axis 48 of elongated body 40. In a particular embodiment, elongated body 40 includes a first passage, e.g., passage 46, and a second passage (not shown) each extending along the first length.

Referring further to FIG. 4, in one embodiment, elongated body 40 includes a core 50 having an inner surface 52 forming passage 46 and an outer surface 54 forming an outer surface of core 50. Core 50 provides rigidity and torque to imaging mini-scope 14 to allow for increased control of navigation, particularly in tighter spaces. In example embodiments, core 50 is formed of a coil made of a suitable material, such as stainless steel, nitinol, nylon monofilament or another plastic material, for example. In alternative embodiments, core 50 may be formed in a braid, mesh, knit, or net construction using suitable materials. In a further alternative embodiment, core 50 is formed as a flexible cannula core having a proximal end portion including a semi-rigid tube, e.g., a hollow cylindrical or non-cylindrical body, made of stainless steel, nitinol or another suitable material, for example, to provide sufficient column strength, and having a distal end portion in a coiled, braided, mesh, net, or knit construction to provide flexibility and deflection. In particular embodiments, a first portion of the coil forming core 50, e.g., a first coil portion at distal end 42, has a first coil pitch and a second portion of the coil forming core 50, e.g., a second coil portion at proximal end 44, has a second coil pitch different than the first coil pitch. In example embodiments, the first coil portion is relatively loosely-pitched for kink resistance and improved deflection, while the second coil portion is relatively tightly-pitched for column strength and rigidity. The coil pitch can vary to adjust or enhance certain characteristics of core 50. For example, in certain embodiments, the coil pitch is tighter at the proximal end portion (i.e., more windings per linear centimeter (cm)) to provide sufficient or additional column strength, while the coil pitch at the distal end portion is looser relative to the coil pitch at the proximal end portion (i.e., less windings per linear cm) to provide more flexibility and deflection. In certain embodiments, imaging mini-scope 14 includes core 50 having a constant coil pitch along a length of elongated body 40 and/or a length of flexible distal tip portion 80, described below. Alternatively, imaging mini-scope 14 may include core 50 having a coil pitch that varies along the length of elongated body 40 and/or the length of flexible distal tip portion 80, depending at least in part on the required flexibility of the component or components. In further embodiments, core 50 may include one layer or a plurality of overlying layers each having a coil, braid, knit, mesh or flexible cannula construction, for example, with a single pitch or multiple pitches. In particular embodiments, core 50 includes a continuous coil with the first coil portion transitioning into second coil portion. In alternative embodiments, core 50 includes a braid, stent, mesh, net, or flexible cannula construction rather than a coil construction.

As shown in FIG. 4, a sheath 56 is positioned about outer surface 54 of core 50. In example embodiments, sheath 56 has a hydrophilic outer surface to facilitate movement of imaging mini-scope 14 within a lumen of the patient. For example, sheath 56 may be made of a suitable lubricious polymer material including, without limitation, a fluoropolymer liner, e.g., as polytetrafluoroethylene (PTFE) or Teflon® material, or another lubricious material such as polyethylene, polyimide, polypropylene, nylon or polyurethane, for smooth movement of imaging mini-scope 14 in the lumen during introduction of imaging mini-scope 14 into the lumen and removal of imaging mini-scope 14 from the lumen. Sheath 56 also protects the internal imaging device and light source of imaging mini-scope 14, as described below.

In example embodiments, elongated body 40 has a first length of 40.0 centimeters (cm) (15.748 inches) to 150.0 cm (59.055 inches) and, more particularly, 50.0 cm (19.685 inches) to 125.0 cm (49.213 inches) and, even more particularly, a first length of 75.0 cm (29.537 inches) to 100.0 cm (39.370 inches) suitable to allow the user to reach the multiple poles of the patient's kidney by transurethral introduction, for example. In alternative embodiments, elongated body 40 may have any suitable length less than 40.0 cm or greater than 150.0 cm. In example embodiments, elongated body 40 has an outer diameter of 0.070 cm (0.028 inches) to 0.150 cm (0.060 inches) and, more particularly, an outer diameter of 0.085 cm (0.033 inches) to 0.140 cm (0.055 inches) and, even more particularly, an outer diameter of 0.0965 cm (0.038 inch) to 0.127 cm (0.050 inch). In alternative embodiments, elongated body 40 may have any suitable outer diameter less than 0.070 cm or greater than 0.150 cm. In a particular embodiment, elongated body 40 is tapered along the first length. For example, elongated body 40 may be tapered in one or more of the following areas or directions: towards or at distal end 42, towards or at proximal end 44, and/or at a midsection of elongated body 40 forming an hourglass shape.

In example embodiments, an imaging device 60 extends through the at least one passage 46. Imaging device 60 includes an imaging sensor 62 disposed at distal end 42 of elongated body 40 and a signal transmission connection 64 coupled to imaging sensor 62. As shown in FIG. 4, in certain embodiments, passage 46 through elongated body 40 is configured to accommodate at least a portion of imaging device 60. More specifically, signal transmission connection 64 extends through passage 46 to operatively couple, e.g., in signal or electronic communication, imaging sensor 62 to an imaging control unit 66, as shown in FIG. 3, disposed at proximal end 44 of elongated body 40 or to another suitable external processing system communicatively coupled to imaging sensor 62 through signal transmission connection 64. Imaging sensor 62 is configured to detect image information and transmit one or more signals indicative of the detected image information to signal transmission connection 64 and signal transmission connection 64 is configured to transmit the one or more signals indicative of the detected image information from imaging sensor 62 to imaging control unit 66.

In example embodiments, imaging device 60 includes a suitable imaging device sized and configured to navigate the tortious passages and multiple poles of the patient's kidney by transurethral introduction. Imaging device 60 may include, for example, a solid state imaging device (SSID), such as a charged coupled device (CCD) camera, having a gradient refractive index (GRIN) lens. The term "solid state imaging device" generally refers to a camera or imaging device having a size approximately equal to or less than the diameter of a bundle of optical fibers. Suitable SSIDs include, for example, charge-injection devices (CID), charge-coupled devices (CCD), complementary metal oxide semiconductor (CMOS) devices, and other miniature-sized imaging devices, including those made from compound semiconductors such as InGaAs, capable of imaging reflected illumination of visible and/or non-visible light. In certain embodiments, the SSID is configured to transmit recorded images to imaging control unit 66 or another external processing system via signal transmission connection 64, disposed within passage 46. In alternative embodiments, the image information is sent via a wireless connection to imaging control unit 66 or the external processing system.

A light source 70 extends through passage 46 and is configured to emit light at distal end 42 of elongated body 40. In example embodiments, passage 46 is configured to accommodate at least a portion of imaging device 60, e.g., signal transmission connection 64, and at least a portion of light source 70, e.g., a flexible optical conductor 72 coupling light source 70 to imaging control device 66. In a particular embodiment, as mentioned above, elongated body 40 includes a first passage configured to accommodate at least a portion of imaging device 60, e.g., signal transmission connection 64, and a second passage configured to accommodate at least a portion of light source 70, e.g., flexible optical conductor 72. In example embodiments, light source 70 includes any light source configured to emit a suitable amount of light at the target site. For example, light source 70 may include a light emitting diode (LED) light source, a fiber optic light source, a laser, light beads, fiber optic beads, or another suitable light source. With elongated body 40 inserted into a patient's lumen, light source 70 emits one or more beams of optical energy that propagates through a flexible optical conductor 72 of light source 70 extending through elongated body 40. Imaging device 60, e.g., imaging sensor 62, can image the illumination reflected by an object during navigation of imaging device 60 through the lumen or at the target site, e.g., interior walls of the lumen or kidney, in response to the beam of optical energy.

As shown in FIG. 3, in example embodiments, image information captured and recorded by imaging device 60 is filtered and processed by imaging control unit 66 or another external processing system, having imaging software 74 for processing and displaying images on a display screen 76 positioned on imaging control unit 66 or an external display operatively coupled to imaging control unit 66 or the external processing system. In example embodiments, imaging control unit 66 or the external processing system controls light source 70 via optical conductor 72.

Referring further to FIGS. 3-5, in example embodiments, imaging mini-scope 14 includes a flexible distal tip portion 80 coupled to distal end 42 of elongated body 40. In a particular embodiment, flexible distal tip portion 80 is removably coupled to elongated body 40. Flexible distal tip portion 80 has a second length less than the first length of elongated body 40. As shown in FIG. 5, for example, imaging sensor 62 is disposed at, e.g., coupled to or at least partially embedded in, flexible distal tip portion 80. Referring further to FIG. 4, imaging sensor 62 is disposed at flexible distal tip portion 80 and signal transmission connection 64 extends through passage 46 of elongated body 40 to operatively couple, e.g., communicatively couple, imaging sensor 62 to imaging control unit 66. In example embodiments, light source 70 is also disposed at, e.g., coupled to or at least partially embedded in, flexible distal tip portion 80. As shown in FIG. 4, light source 70 and/or optical conductor 72 at least partially extend through passage 46 of elongated body 40 to operatively couple, e.g., communicatively couple, light source 70 to imaging control unit 66 or another external processing system.

FIG. 6 is a sectional side view of an example imaging mini-scope 14 with a flexible distal tip portion 80. In an example embodiment, imaging mini-scope 14 includes flexible distal tip portion 80 coupled to distal end 42 of elongated body 40, e.g., a flexible cannula made of nitinol, stainless steel or another suitable material for column strength. Flexible distal tip portion 80 includes a coil 82 or a braid for deflection and kink-resistant flexibility during navigation of imaging mini-scope 14 through a lumen, for example.

In example embodiments, flexible distal tip portion 80 is configured to deflect in a plurality of directions including, for example, a first direction and a second direction different from the first direction. In certain embodiments, flexible distal tip portion 80 is configured to deflect at least 180°. Flexible distal tip portion 80 is coupled to distal end 42 of elongated body 40 and is configured to navigate through tight spaces and prevent perforation of the human lumen or vessel in which imaging mini-scope 12 is positioned. In example embodiments, flexible distal tip portion 80 has a length of 2.0 cm (0.787 inches) to 15.0 cm (5.901 inches) and, more particularly, 4.0 cm (1.575 inches) to 12.0 cm (4.724 inches) and, even more particularly, a length of 5.0 cm (1.969 inches) to 8.0 cm (3.150 inches). In alternative embodiments, flexible distal tip portion 80 may have any suitable length less than 2.0 cm or greater than 15.0 cm. Further, flexible distal tip portion 80 may have any suitable outer diameter, such as an outer diameter similar or equal to the outer diameter of elongated body 40, as described above. Flexible distal tip portion 80 includes a loosely-pitched coil portion, i.e., the first coil portion at distal end 42 of elongated body 40, as described above, to facilitate controllable deflection of flexible distal tip portion 80. In alternative embodiments, core 50 at or near flexible distal tip portion 80 may be formed in a braid, mesh, knit, or net construction using suitable materials. In a further alternative embodiment, core 50 at or near flexible distal tip portion 80 is formed as at least a portion of a flexible cannula core, as described above. In a particular embodiment, an outer surface of flexible distal tip portion is tapered.

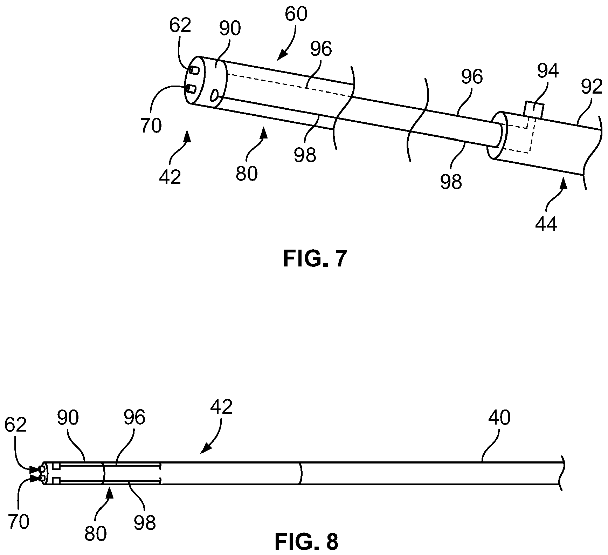

Referring now to FIG. 7, in certain example embodiments, imaging mini-scope 14 includes a deflection band 90 operatively coupled to flexible distal tip portion 80 to urge or control deflection of flexible distal tip portion 80 to direct imaging sensor 62 in a desired direction for observation of the target site. As shown in FIGS. 3-7, imaging mini-scope 12 includes a handle 92 disposed at proximal end 44 of elongated body 40. Handle 92 can be made of any suitable material including, without limitation, a plastic material such as polycarbonate, which is strong enough to withstand the forces required to deflect flexible distal tip portion 80. Further, handle 92 has a suitable ergonomic design to deflect flexible distal tip portion 80 and hold flexible distal tip portion 80 in a deflected position. Handle 92 is operatively coupled to deflection band 90 to move flexible distal tip portion 80 in one of a plurality of directions, for example, a first direction or a second direction different from the first direction. FIG. 5 shows flexible distal tip portion 80 in a deflected position in a first direction, for example. More specifically, handle 92 includes a deflection actuator 94 coupled to a first pull wire 96 coupled between deflection actuator 94 and deflection band 90. Deflection actuator 94 is configured to control movement of first pull wire 96 to urge flexible distal tip portion 80 to move, i.e., deflect, in one or more directions, e.g., a first direction, for example. Deflection actuator 94 is also configured to control movement of a second pull wire 98 coupled between deflection actuator 94 and deflection band 90 to move second pull wire 98 to urge flexible distal tip portion 80 to move, i.e., deflect, in one or more directions, e.g., a second direction different than the first direction. In example embodiments, deflection actuator 94 is coupled to any suitable number of pull wires to control movement of flexible distal tip portion 80 in any of a plurality of directions, as desired. As shown in FIG. 7, first pull wire 96 is coupled to a first side of deflection band 90 and second pull wire 98 is coupled to an opposite second side of deflection band 90 in example embodiments. As such, deflection actuator 94 moves first pull wire 96 and/or second pull wire 98 to controllably deflect flexible distal tip portion 80 to a desired position in one of a plurality of directions. In example embodiments, deflection band 90 is formed of a suitable material, such as stainless steel, a suitable plastic material, or another suitable metal material, for example, and first and second pull wires 96, 98 are made of stainless steel, nitinol, or a monofilament, for example, to facilitate deflection capabilities. In certain embodiments, the pull wires are cannula crimped or glued to deflection band 90 or flexible distal end portion 80, e.g., inside flexible distal tip portion 80, to anchor the pull wires to deflection band 90 and/or flexible distal end portion 80. Alternatively, at least a portion of each pull wire can be at least partially embedded in deflection band 90 or flexible distal end portion 80. For example, a knot may be formed in a distal portion of each pull wire and the knot at least partially embedded in flexible distal tip portion 80 to anchor each pull wire to deflection band 90. In other embodiments, the pull wires can be operatively coupled to deflection band 90 using any suitable technique.

In one embodiment, as shown in FIG. 8, deflection band 90 is formed of a rigid plastic material or another suitable material, which is coupled to flexible distal tip portion 80. Flexible distal tip portion 80 is coupled to distal end 42 of elongated body 40, formed of a rigid plastic material. In this embodiment, a distal portion of each pull wire, e.g., first pull wire 96 and second pull wire 98, is at least partially embedded in deflection band 90 to anchor each pull wire to deflection band 90.

Referring again to FIGS. 3-6, imaging control unit 66 is disposed at proximal end 44 of elongated body 40 and operatively coupled to imaging device 60, e.g., coupled in operational controller communication with imaging device 60. In an example embodiment, imaging control unit 66 is integrated with or coupled to handle 92. Imaging control unit 66 is configured to control operation of imaging device 60 and, particularly imaging sensor 62, as well as light source 70. Imaging control unit 66 is communicatively coupled to imaging sensor 62 and configured to transmit signals to and receive signals from imaging sensor 62 via signal transmission connection 64, e.g., one or more signals indicative of imaging information detected by imaging sensor 62. Imaging control unit 66 is also configured to control the imaging detection of imaging sensor 62, e.g., a direction in which imaging sensor 62 is positioned. In certain embodiments, imaging control unit 66 is also configured to control operation of light source 70. For example, imaging control unit 66 may be configured to adjust parameters of the light emitted by light source 70, such as an amount or an intensity of the emitted light and/or a direction of the emitted light. In alternative embodiments, imaging sensor 62 and imaging control unit 66 may communicate using other suitable communication protocol including, for example, wireless communication. In certain embodiments, imaging control unit 66 is coupled to an external computer or processing system (not shown) for processing the imaging information that imaging control unit 66 receives from imaging sensor 62 through transmission connection 64 to generate one or more images of the target site.

In example embodiments, the imaging mini-scope is placed transurethral, inserted through the patient's urethra and into the patient's bladder, for example. The imaging mini-scope is navigated through the UVJ and into the ureter. As the mini-scope navigates up the ureter, the imaging mini-scope visualizes the inner wall of the ureter and can be used diagnostically to identify strictures, stones, etc. Visualization, as well as the deflection properties of the flexible distal tip portion and the rigid core of the elongated body allows the imaging mini-scope to navigate past the stricture, for example, and move up into the patient's kidney. If, for example, a stone in the ureter or the kidney needs to be removed, a rapid exchange dilator with an access sheath is placed on the imaging mini-scope in front of the handle of the imaging mini-scope and inserted into the body lumen and up to the source of the stone burden. Once the flexible distal tip portion of the imaging mini-scope reaches the source of the stone burden, the imaging mini-scope can be removed from the access sheath, to release the imaging mini-scope from the dilator. Devices or instruments for removing the stone are then introduced through the access sheath and visualized via the imaging mini-scope decoupled from the access sheath. The example imaging mini-scope and/or the described system may be suitable for use in other procedures or applications including, without limitation, interventional radiology, aortic intervention, and peripheral intervention, for example.

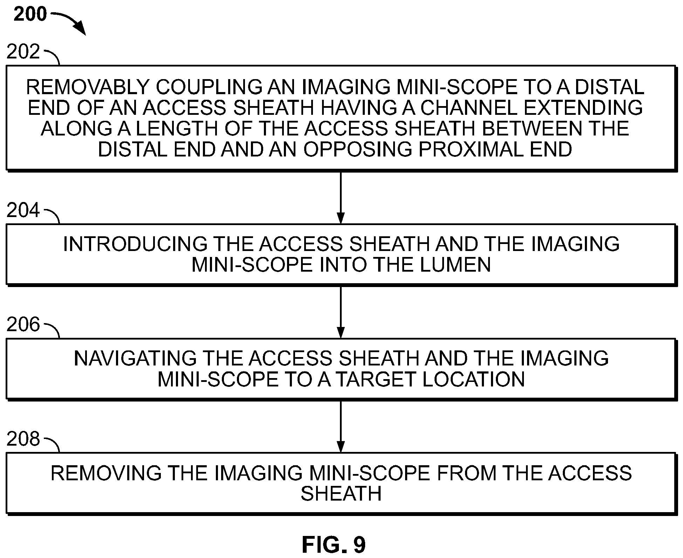

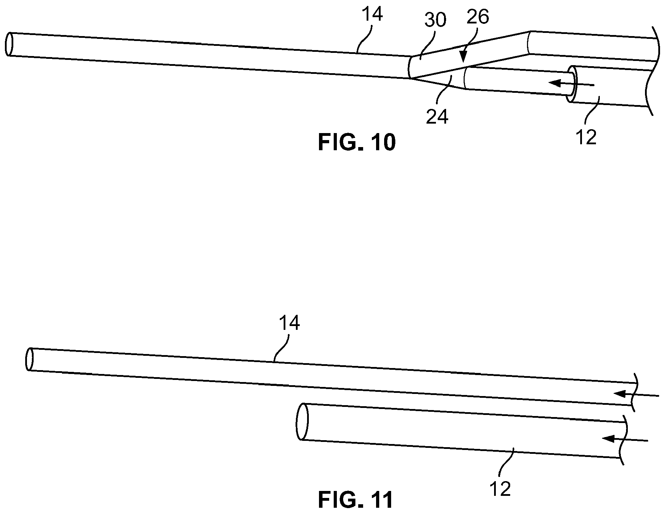

FIG. 9 is a flow diagram of an example method 200 for introducing an imaging mini-scope, such imaging mini-scope 14 as shown in FIGS. 1-7, into a lumen of a patient. FIGS. 10 and 11 are schematic views of a portion of system 10 including an access sheath, such as access sheath 12, and an imaging mini-scope, such as imaging mini-scope 14, removably coupled to access sheath 12 during various steps of the example method 200 illustrated in FIG. 9.

At step 202, an imaging mini-scope is removably coupled to a distal end of an access sheath having a channel extending along a length of the access sheath between the distal end and an opposing proximal end. In a particular embodiment, at least a portion of the imaging mini-scope is positioned in a passage of a dilator of the access sheath. The dilator is disposed at a distal end of the access sheath having a channel extending along a length of the access sheath between the distal end and an opposing proximal end. The passage of the dilator is coaxial with the channel. The dilator has a longitudinal slit intersecting the passage. At step 204, the access sheath and the imaging mini-scope coupled to the access sheath are introduced into the lumen. The access sheath and the imaging mini-scope are navigated 206 to a target site. At or near the target site, the imaging mini-scope is removed 208 from the access sheath. In a particular embodiment, removing the imaging mini-scope from the access sheath includes removing the imaging mini-scope from the passage through the longitudinal slit. For example, referring to FIGS. 10 and 11, in an example embodiment, at the target site, access sheath 12 is moved distally towards dilator 24 to urge imaging mini-scope 14 from passage 26 through longitudinal slit 30 to remove imaging mini-scope 14 from access sheath 12. In certain embodiments, imaging mini-scope 14 may be pre-loaded onto access sheath 12 before access sheath 12 and mini-scope 14 are introduced into the lumen or, in alternative embodiments, imaging mini-scope 14 may be coupled to access sheath 12 after access sheath 12 and mini-scope 14 are independently introduced into the lumen.

Imaging control unit 66 may be implemented as any of a number of different types of electronic devices. Some examples of imaging control unit 66 may include tablet computing devices, mobile devices, laptop and netbook computing devices or any other device capable of connecting with image device 60 and/or light source 70 and including a processor and memory for controlling image device 60 and/or light source 70 according to the techniques described herein.

In a very basic configuration, imaging control unit 66 includes, or accesses, components such as at least one control logic circuit, central processing unit, or processor, and one or more computer-readable media. Each processor may itself comprise one or more processors or processing cores. For example, each processor can be implemented as one or more microprocessors, microcomputers, microcontrollers, digital signal processors, central processing units, state machines, logic circuitries, and/or any devices that manipulate signals based on operational instructions. In some cases, the processor may be one or more hardware processors and/or logic circuits of any suitable type specifically programmed or configured to execute the algorithms and processes described herein. The processor can be configured to fetch and execute computer-readable instructions stored in a computer-readable media or other computer-readable media.

Depending on the configuration of imaging control unit 66, computer-readable media may be an example of tangible non-transitory computer storage media and may include volatile and nonvolatile memory and/or removable and non-removable media implemented in any type of technology for storage of information such as computer-readable instructions, data structures, program modules or other data. The computer-readable media may include, but is not limited to, RAM, ROM, EEPROM, flash memory or other computer readable media technology, CD-ROM, digital versatile disks (DVD) or other optical storage, magnetic cassettes, magnetic tape, solid-state storage and/or magnetic disk storage. Further, in some cases, imaging control unit 66 may access external storage, such as RAID storage systems, storage arrays, network attached storage, storage area networks, cloud storage, or any other medium that can be used to store information and that can be accessed by the processor directly or through another computing device or network. Accordingly, computer-readable media may be computer storage media able to store instructions, modules or components that may be executed by the processor.

Computer-readable media may be used to store and maintain any number of functional components that are executable by the processor. In some implementations, these functional components comprise instructions or programs that are executable by the processor and that, when executed, implement operational logic for performing the actions attributed above to imaging control unit 66. Functional components of imaging control unit 66 stored in the computer-readable media may include the operating system and a user interface module for controlling and managing various functions of imaging device 60 and/or light source 70, and for generating one or more user interfaces on imaging control unit 66.

Imaging control unit 66 may further include one or more communication interfaces, which may support both wired and wireless connection to various networks, such as cellular networks, radio, Wi-Fi networks, close-range wireless connections, near-field connections, infrared signals, local area networks, wide area networks, the Internet, and so forth. The communication interfaces may further allow a user to access storage on or through another device, such as a remote computing device, a network attached storage device, cloud storage, or the like.

Imaging control unit 66 may further be equipped with one or more various input/output (I/O) components. Such I/O components may include a touchscreen and various user controls (e.g., buttons, a joystick, a keyboard, a keypad, etc.), a haptic or tactile output device, connection ports, physical condition sensors, and so forth. For example, the operating system of imaging control unit 66 may include suitable drivers configured to accept input from a keypad, keyboard, or other user controls and devices included as I/O components. Additionally, imaging control unit 66 may include various other components that are not shown, examples of which include removable storage, a power source, such as a battery and power control unit, a PC Card component, and so forth.

Various instructions, methods and techniques described herein may be considered in the general context of computer-executable instructions, such as program modules stored on computer storage media and executed by the processors herein. Generally, program modules include routines, programs, objects, components, data structures, etc., for performing particular tasks or implementing particular abstract data types. These program modules, and the like, may be executed as native code or may be downloaded and executed, such as in a virtual machine or other just-in-time compilation execution environment. Typically, the functionality of the program modules may be combined or distributed as desired in various implementations. An implementation of these modules and techniques may be stored on computer storage media or transmitted across some form of communication.

Embodiments of the disclosure can be described in view of the following clauses:
1. An imaging mini-scope, comprising: an elongated body having a first length, the elongated body including at least one passage extending along the first length; a flexible distal tip portion coupled to the elongated body; an imaging device including an imaging sensor disposed at the flexible distal tip portion and a signal transmission connection extending through the at least one passage to connect the imaging sensor to a processing system, wherein the imaging sensor is configured to detect image information and the signal transmission connection is configured to transmit one or more signals indicative of the detected image information from the imaging sensor to the processing system; and a light source disposed at the flexible distal tip portion, the light source extending through the at least one passage.
2. The imaging mini-scope of clause 1, wherein the at least one passage includes a first passage extending along the first length, the signal transmission connection positioned within the first passage, and a second passage extending along the first length, the light source positioned within the second passage.
3. The imaging mini-scope of clauses 1 or 2, wherein the flexible distal tip portion is configured to deflect in a plurality of directions.
4. The imaging mini-scope of any of clauses 1-3, wherein the flexible distal tip portion comprises a first coil portion having a first coil pitch and the elongated body comprises a second coil portion having a second coil pitch different from the first coil pitch.
5. The imaging mini-scope of any of clauses 1-4, wherein the flexible distal tip portion has a second length of 2.0 centimeters (cm) to 15.0 cm.
6. The imaging mini-scope of any of clauses 1-5, further comprising: a deflection band coupled to the flexible distal tip portion; and a first pull wire coupled to the deflection band, the first pull wire movable to facilitate deflecting the flexible distal tip portion in a first direction.
7. The imaging mini-scope of clause 6, further comprising a second pull wire coupled to the deflection band, the second pull wire movable to facilitate deflecting the flexible distal tip portion in a second direction different from the first direction.
8. The imaging mini-scope of clause 7, further comprising a handle coupled to the elongated body, the handle including a deflection actuator coupled to each of the first pull wire and the second pull wire.
9. The imaging mini-scope of any of clauses 1-8, wherein the elongated body has a first length of 40.0 centimeters (cm) to 150.0 cm.
10. The imaging mini-scope of any of clauses 1-9, wherein the elongated body has an outer diameter of 0.070 cm to 0.150 cm.
11. The imaging mini-scope of any of clauses 1-10, wherein an outer surface of at least one of the flexible distal tip portion or the elongated body is tapered.
12. The imaging mini-scope of any of clauses 1-11, wherein the flexible distal tip portion is removably coupled to the elongated body.
13. The imaging mini-scope of any of clauses 1-12, wherein the elongated body comprises: a core having an inner surface forming the at least one passage and an outer surface; and a sheath positioned about the outer surface of the core, the sheath having a hydrophilic outer surface.
14. A system, comprising: an access sheath having a channel extending along a first length of the access sheath between a first distal end and an opposing first proximal end; a dilator disposed at the first distal end, the dilator comprising a passage coaxial with the channel and a longitudinal slit intersecting the passage; and an imaging mini-scope at least partially positioned in the passage and removable from the passage through the longitudinal slit, the imaging mini-scope comprising: an elongated body having at least one passage extending along a second length of the elongated body between a second distal end and an opposing second proximal end; an imaging device extending through the at least one passage, the imaging device including an imaging sensor disposed at the second distal end and a signal transmission connection coupled to the imaging sensor; and a light source extending through the at least one passage, the light source configured to emit light at the second distal end.
15. The system of clause 14, wherein the at least one passage further comprises: a first passage extending along the second length, the first passage configured to accommodate at least a portion of the imaging device; and a second passage extending along the second length, the second passage configured to accommodate at least a portion of the light source.
16. The system of clauses 14 or 15, wherein the imaging mini-scope further comprises a flexible distal tip portion coupled to the second distal end of the elongated body, the flexible distal tip portion movable in a first direction and a second direction different than the first direction, wherein at least a portion of the imaging sensor and at least a portion of the light source are embedded in the flexible distal tip portion.
17. The system of clause 16, further comprising: a deflection band operatively coupled to the flexible distal tip portion; and a handle disposed at the second proximal end, the handle operatively coupled to the deflection band to move the flexible distal tip portion in one of the first direction or the second direction.
18. The system of clause 17, wherein the handle comprises: a deflection actuator; a first pull wire coupled between the deflection actuator and the deflection band, wherein the deflection actuator is configured to move the first pull wire to deflect the flexible distal tip portion in the first direction; and a second pull wire coupled between the deflection actuator and the deflection band, wherein the deflection actuator is configured to move the second pull wire to deflect the flexible distal tip portion in the second direction.
19. The system of any of clauses 14-18, further comprising an imaging control unit disposed at the second proximal end, the imaging control unit coupled in operational control communication with the imaging device for controlling operation of the imaging device and receiving one or more signals from the imaging device indicative of imaging information detected by the imaging sensor.
20. A method for introducing an imaging mini-scope into a lumen of a human, the method comprising: removably coupling an imaging mini-scope to a distal end of an access sheath having a channel extending along a length of the access sheath between the distal end and an opposing proximal end; introducing the access sheath and the imaging mini-scope into the lumen; navigating the access sheath and the imaging mini-scope to a target location; and removing the imaging mini-scope from the access sheath.
21. The method of clause 20, wherein removably coupling an imaging mini-scope to a distal end of an access sheath comprises positioning at least a portion of an imaging mini-scope in a passage of a dilator, the dilator disposed at the distal end of an access sheath, the passage of the dilator coaxial with the channel, the dilator having a longitudinal slit intersecting the passage; and wherein removing the imaging mini-scope from the access sheath comprises removing the imaging mini-scope from the passage through the longitudinal slit.

Although the subject matter has been described in language specific to structural features and/or methodological acts, it is to be understood that the subject matter defined in the appended claims is not necessarily limited to the specific features or acts described. Rather, the specific features and acts are disclosed as illustrative forms of implementing the claims.

One skilled in the art will realize that a virtually unlimited number of variations to the above descriptions are possible, and that the examples and the accompanying figures are merely to illustrate one or more examples of implementations.

It will be understood by those skilled in the art that various other modifications can be made, and equivalents can be substituted, without departing from claimed subject matter. Additionally, many modifications can be made to adapt a particular situation to the teachings of claimed subject matter without departing from the central concept described herein. Therefore, it is intended that claimed subject matter not be limited to the particular embodiments disclosed, but that such claimed subject matter can also include all embodiments falling within the scope of the appended claims, and equivalents thereof.

In the detailed description above, numerous specific details are set forth to provide a thorough understanding of claimed subject matter. However, it will be understood by those skilled in the art that claimed subject matter can be practiced without these specific details. In other instances, methods, devices, or systems that would be known by one of ordinary skill have not been described in detail so as not to obscure claimed subject matter.

Reference throughout this specification to "one embodiment" or "an embodiment" can mean that a particular feature, structure, or characteristic described in connection with a particular embodiment can be included in at least one embodiment of claimed subject matter. Thus, appearances of the phrase "in one embodiment" or "an embodiment" in various places throughout this specification are not necessarily intended to refer to the same embodiment or to any one particular embodiment described. Furthermore, it is to be understood that particular features, structures, or characteristics described can be combined in various ways in one or more embodiments. In general, of course, these and other issues can vary with the particular context of usage. Therefore, the particular context of the description or the usage of these terms can provide helpful guidance regarding inferences to be drawn for that context.

The Abstract of this specification is repeated here as follows: An imaging mini-scope for use with an endoscope system includes an elongated body having a first length. The elongated body includes at least one passage extending along the first length. A flexible distal tip portion is coupled to the elongated body. An imaging device includes an imaging sensor disposed at the flexible distal tip portion and a signal transmission connection extending through the at least one passage to connect the imaging sensor to a processing system. The imaging sensor is configured to detect image information and the signal transmission connection is configured to transmit one or more signals indicative of the detected image information from the imaging sensor to the processing system. A light source is disposed at the flexible distal tip portion. The light source extends through the at least one passage.

## Claims

1. An imaging mini-scope, comprising:
an elongated body having a first length, the elongated body including at least one passage extending along the first length;
a flexible distal tip portion coupled to the elongated body;
an imaging device including an imaging sensor disposed at the flexible distal tip portion and a signal transmission connection extending through the at least one passage to connect the imaging sensor to a processing system, wherein the imaging sensor is configured to detect image information and the signal transmission connection is configured to transmit one or more signals indicative of the detected image information from the imaging sensor to the processing system; and
a light source disposed at the flexible distal tip portion, the light source extending through the at least one passage.

2. The imaging mini-scope of claim 1, wherein the at least one passage includes a first passage extending along the first length, the signal transmission connection positioned within the first passage, and a second passage extending along the first length, the light source positioned within the second passage.

3. The imaging mini-scope of claim 1 or claim 2, wherein the flexible distal tip portion is configured to deflect in a plurality of directions.

4. The imaging mini-scope of any one of the preceding claims, wherein the flexible distal tip portion comprises a first coil portion having a first coil pitch and the elongated body comprises a second coil portion having a second coil pitch different from the first coil pitch.

5. The imaging mini-scope of any one of the preceding claims, wherein the flexible distal tip portion has a second length of 2.0 centimeters (cm) to 15.0 cm.

6. The imaging mini-scope of any one of the preceding claims, further comprising:
a deflection band coupled to the flexible distal tip portion; and
a first pull wire coupled to the deflection band, the first pull wire movable to facilitate deflecting the flexible distal tip portion in a first direction.

7. The imaging mini-scope of claim 6, further comprising a second pull wire coupled to the deflection band, the second pull wire movable to facilitate deflecting the flexible distal tip portion in a second direction different from the first direction.

8. The imaging mini-scope of claim 7, further comprising a handle coupled to the elongated body, the handle including a deflection actuator coupled to each of the first pull wire and the second pull wire.

9. The imaging mini-scope of any one of the preceding claims, wherein the elongated body has a first length of 40.0 centimeters (cm) to 150.0 cm and the elongated body has an outer diameter of 0.070 cm to 0.150 cm.

10. The imaging mini-scope of any one of the preceding claims, wherein an outer surface of at least one of the flexible distal tip portion or the elongated body is tapered.

11. The imaging mini-scope of any one of the preceding claims, wherein the flexible distal tip portion is removably coupled to the elongated body.

12. The imaging mini-scope of any one of the preceding claims, wherein the elongated body comprises:
a core having an inner surface forming the at least one passage and an outer surface; and
a sheath positioned about the outer surface of the core, the sheath having a hydrophilic outer surface.

13. The imaging mini-scope of any one of the preceding claims, further comprising an imaging control unit coupled in operational control communication with the imaging device for controlling operation of the imaging device and receiving one or more signals from the imaging device indicative of imaging information detected by the imaging sensor.

14. An assembly for introducing an imaging mini-scope into and providing access to a lumen of a human, the assembly comprising:
the imaging mini-scope of any of the preceding claims;
an access sheath having a channel extending along a length of the access sheath between the distal end and an opposing proximal end, the access sheath having means for removably coupling the imaging mini-scope to a distal end of the access sheath enabling
the introduction of the access sheath and the imaging mini-scope into the lumen;
the navigation of the access sheath and the imaging mini-scope to a target location; and
the removal of the imaging mini-scope from the access sheath.

15. The assembly of claim 14, wherein the means for removably coupling an imaging mini-scope to a distal end of an access sheath comprises a passage of a dilator, the dilator disposed at the distal end of an access sheath, the passage of the dilator coaxial with the channel, at least a portion of the imaging mini-scope being positionable in the passage, the dilator having a longitudinal slit intersecting the passage enabling the removal of the imaging mini-scope from the access sheath by removing the imaging mini-scope from the passage through the longitudinal slit.
